(19) Europäisches Patentamt
European Patent Office
Office européen des brevets

(11) Publication number: **0 441 563 B1**

(12) **EUROPEAN PATENT SPECIFICATION**

(45) Date of publication of patent specification: **24.05.95**

(51) Int. Cl.[6]: **C07C 271/22**, C08F 18/00, C11D 3/37

(21) Application number: **91300871.0**

(22) Date of filing: **04.02.91**

(54) **Detergent compositions containing vinyl carbamate compounds.**

(30) Priority: **06.02.90 US 475660**
**06.02.90 US 475666**

(43) Date of publication of application:
**14.08.91 Bulletin 91/33**

(45) Publication of the grant of the patent:
**24.05.95 Bulletin 95/21**

(84) Designated Contracting States:
**CH DE ES FR GB IT LI NL SE**

(56) References cited:
**EP-A- 0 109 044**
**EP-A- 0 125 208**
**EP-A- 0 171 362**
**FR-A- 2 502 630**
**FR-A- 2 612 521**
**US-A- 3 711 458**
**US-A- 4 382 949**

(73) Proprietor: **UNILEVER PLC**
**Unilever House**
**Blackfriars**
**London EC4P 4BO (GB)**
(84) Designated Contracting States:
**GB**

(73) Proprietor: **UNILEVER N.V.**
**Weena 455**
**NL-3013 AL Rotterdam (NL)**

(84) Designated Contracting States:
**CH DE ES FR IT LI NL SE**

(72) Inventor: **Wu, Shang Ren**
**Unilever Research US, Inc,**
**45 River Road**
**Edgewater, NJ 07020 (US)**
Inventor: **Garofalo, Albert**
**Unilever Research US, Inc,**
**45 River Road**
**Edgewater, NJ 07020 (US)**

(74) Representative: **Fransella, Mary Evelyn et al**
**Unilever PLC**
**Patent Division**
**Colworth House**
**Sharnbrook**
**Bedford MK44 1LO (GB)**

EP 0 441 563 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to polymeric vinyl carbamates which are effective chelating agents and useful in detergent compositions. These polymers may also be used as antiredeposition agents, dispersants, scale inhibitors, bleach stabilising agents and in a variety of other applications which require hardness sequestration or crystal modification.

### BACKGROUND AND PRIOR ART

Builders are desirable ingredients in powdered detergent formulations, which optimise the effectiveness of surfactants by sequestering calcium, magnesium and other "hardness" ions present in the wash water that adversely affect detergency.

The manner in which detergent builders improve the cleaning powers of detergent compositions is related to a combination of factors such as emulsification of soil particles, solubilisation of water-insoluble materials, promotion of oil suspension in the wash water so as to retard soil redeposition, sequestration of metallic tons, and the like.

Phosphates, such as tripolyphosphates and pyrophosphates, are widely used as builders due to their excellent ability to sequester "hardness" ions. However, the effect of phosphates upon the eutrophication of lakes and streams has been questioned and their use in detergent compositions has been subject to government scrutiny and regulation. Alternatives for phosphates are widely used by detergent formulators as builders in detergent formulations. Compositions and materials change frequently as formulators attempt to improve cleaning performance while offering greater convenience in handling at lower material cost. The industry has made substantial efforts to find suitable substitutes for phosphates, however, all have one or more drawbacks that offset their value in the formulations.

While polymeric carboxylates have been found to be suitable builders, few have been found to be biodegradable, indeed, few synthetic polymers have been found to be biodegradable. One method of improving the biodegradability of synthetic polymers has been to incorporate hydrolysable linkages within the main polymer backbone. This approach, however, usually does not produce high molecular weight polymers. A second method of improving biodegradability is a method of this invention, namely to couple relatively small biodegradable carboxylates to a polymer backbone which will, through hydrolysable linkages, break down to form polyvinyl alcohol (PVA) or a PVA-like copolymer. Upon hydrolysis, the components are then expected to be biodegradable.

The polymeric builders used according to the present invention contain carbamate linkages with environmentally acceptable amino acids. While not wishing to be bound by theory, it is theorised that the carbamate linkages will break down in the environment to polyvinyl alcohol plus the corresponding amino acid, all of which are reported to be relatively environmentally acceptable. Further, the electrostatic and site specific charge interactions of the compounds of this invention contribute to the builder efficiency.

The polymers of the invention are effective "hardness" ion sequestering agents which may be employed in detergent compositions as a replacement, in whole or in part, for phosphate builders.

FR 2 612 521A (Centre Nationale de la Recherche Scientifique) discloses chelating polymers similar to those of the invention, useful in industry, inter alia for detergents, but primarily intended for the medical field. Vinyl carbamate polymers are also disclosed in FR 2 502 630A (Societe Nationale des Poudres et Explosifs), and US 3 711 458 and US 3 835 109 (Olofson/Research Corporation) disclose processes for peptide synthesis employing a vinyloxy carbonyl group as a protecting group.

US 3 923 742 (Haschke) discloses a process for producing a polycarboxylate phosphate substitute said to be readily biodegradable. US 4 559 159 (Denzinger et al) discloses water-soluble copolymers for use with detergents.

### DEFINITION OF THE INVENTION

The present invention provides a detergent composition comprising from 0.5% to 98% of a surfactant, and further comprising from 2% to 99.5% of a polymeric compound having substantial water solubility and a log $K_{Ca}2+$ value greater than 4, the polymeric compound being a homo- or copolymer comprising from 5 to 100% of units of an N-vinyloxycarbonyl amino acid or salt selected from

N-vinyloxycarbonyl aspartic acid,
N-vinyloxycarbonyl glutamic acid,
N-vinyloxycarbonylglycine,
N-vinyloxycarbonylalanine,

N-vinyloxycarbonylaminobutyric acid,
N-vinyloxycarbonylcysteine,
N-vinyloxycarbonylornithine,
N-vinyloxycarbonyliminodiacetic acid,
and alkali metal or ammonium salts thereof,
and optionally from 0 to 95% of units of a comonomer which is selected from unsaturated carboxylic acids and their salts, esters and derivatives; and unsaturated dicarboxylic acids and their salts, esters, anhydrides and derivatives.

## DETAILED DESCRIPTION OF THE INVENTION

The polymers used according to the invention fall within the general formula V:

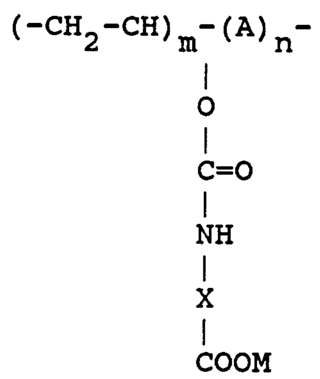

(V)

wherein m represents the number of repeating units of the vinyl carbamate monomer and is at least 1; n represents the number of repeating units of the comonomer and is 0 or an integer of at least 1; M is hydrogen, $C_{1-4}$ alkyl or a cation which forms a substantially water-soluble salt with said polymeric compound at ambient temperatures; NH-X-COOM is an amino acid moiety; and A represents the comonomer.

The vinyl carbamate polymers used according to the invention may be prepared from the corresponding vinyl carbamate monomers by either free radical homopolymerisation or by copolymerisation with one or more other ethylenically unsaturated polymerisable comonomers. Thus, polymers containing two, three or even more comonomers may be employed.

A first preferred class of polymers within the formula V is represented by the formula VI:

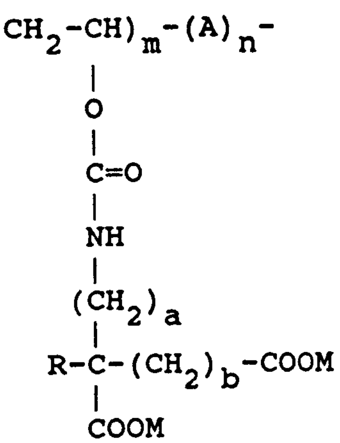

(VI)

wherein m, n, A, and M have the meanings given above; a and b are independently 0, 1 or 2; R is hydrogen or methyl.

Specifically, the polymers within this class contain units of N-vinyloxycarbonyl aspartic acid or N-vinyloxycarbonyl glutamic acid, or salts thereof.

A second preferred class of polymers within the formula V is represented by the formula VII:

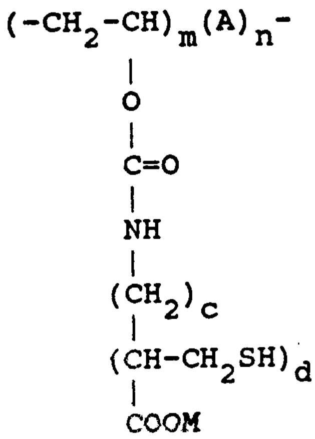

(VII)

wherein m, n, A, and h have the meanings given previously; c is 0 or an integer of 1 to 4; d is 0 to 1; when d is 1, c is 0.

Specifically, the polymers within this class contain units of N-vinyloxycarbonyl glycine, N-vinyloxycarbonylaminobutyric acid, or N-vinyloxycarbonyl cysteine, or salts thereof.

A third preferred class of polymers is represented by general formula VIII:

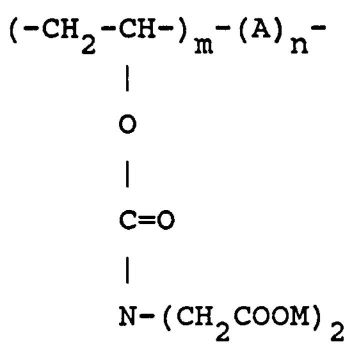

(VIII)

wherein M, A, m and n are as defined above.

Specifically, the polymers within this class contain units of N-vinyloxycarbonyl iminodiacetic acid or salts thereof.

A fourth preferred class of polymers contain units of N-vinyloxycarbonyl alanine or N-vinyloxycarbonyl ornithine; or salts thereof.

The comonomer

The comonomer providing the units A is an ethylenically unsaturated compound, selected from ethylenically unsaturated carboxylic acids and their salts, esters and derivatives, such as acrylic acid, methacrylate, methyl methacrylate, crotonic acid, N-methylacrylyl-D-glucosamine, vinyl benzoic acid, vinylacetic acid, methyl acrylate, itaconic acid, acylamide and methyl acrylamide; and ethylenically unsaturated dicarboxylic acids and their salts, esters, anhydrides and derivatives such as fumaric acid, maleic acid and maleic anhydride.

Those skilled in the art will recognise that the preferred selection of comonomers will be those comonomers best suited to the polymers' intended use. Thus, for detergent use, comonomers with known effectiveness as "hardness" ion sequestrants or as detergent builders are selected. Other factors, such as cost and detergent formulation compatibility will also guide comonomer selection.

Preferred comomomers are acrylic acid and maleic acid.

Optionally, polyunsaturated polymerisable comonomers may also be present in small amounts, ie up to 5% by weight. Such comonomers include those polyethylenically-unsaturated monomers such as lower

alkenyl lower alkenoates, for example, vinyl crotonate, allyl acrylate, allyl methacrylate; di-lower alkenyl alkanedioates, for example, diallyl maleate, divinyl adipate, diallyl adipate; di-lower alkenyl benzenedicarboxylates, for example diallyl phthalate; lower alkanediol di-lower alkenoates, for example ethylene glycol diacrylate, ethylene glycol dimethacrylate, butanediol dimethacrylate; lower alkylene bis-acrylamides and lower alkylene bis-methacrylamides, for example, methylene bis-acrylamide; triallyl cyanurate, etc.

Batch, semi-batch or slow addition methods may be used to prepare the homopolymers or copolymers used in the invention. In accordance with either the batch or semi-batch procedures, the initiator(s), any optional comonomers and the novel carbamate monomer are polymerised in aqueous medium under pressures not exceeding 100 atmospheres in the presence of a catalyst, the aqueous system being maintained by a suitable buffering agent at a pH of 2 to 10, the catalyst being added incrementally or continuously. Suitable as polymerisation catalysts are the water-soluble free-radical formers generally used in polymerisation, such as azobisisobutyronitrile (AIBN), hydrogen peroxide, sodium persulphate, potassium persulphate and ammonium persulphate, as well as tert-butyl hydroperoxide, in amounts of 0.01 to 15% by weight, preferably 0.01 to 1% by weight based on the total amount of monomer(s). The free-radical formers can be charged in the aqueous solution or added during the polymerisation in doses.

The polymerisation may be carried out at a pH of between 2 and 10, preferably between 3 and 5. In order to maintain the pH range, customary buffer systems may be used. For example, alkali-metal acetates, alkali-metal carbonates, alkali-metal phosphates and the like. Polymerisation regulators, such as mercaptans, aldehydes, chloroform, methylene chloride, and trichloroethylene can also be added in some cases.

The polymers used according to the invention contain at least 5% of vinyl carbamate units. Since homopolymers may be used, the actual vinyl carbamate unit content is therefore preferably from 5 to 100%. The polymers may be copolymers, terpolymers or other higher combinations of polymerisable comonomers. In this case, the polymer contains not only vinyl carbamate units but also other repeating units derived from ethylenically unsaturated comonomers, in order to vary the properties of the polymer and particularly its solubility.

The polymers used according to the present invention suitably have a molecular weight of from 1000 to 750000, and preferably of 5000 to 400000.

The molecular weight of the polymers can be controlled by the usual methods, for example, by varying the concentration of monomer(s), initiator(s) and chain-transfer agent(s).

For detergent and sequestrant use, the polymers must be sufficiently water-soluble to function as a builder to sequester hardness ions such as calcium, magnesium and the like.

The polymers also have a log $K_{Ca}2+$ value greater than 4.

The detergent compositions

Polymers of the present invention, when in substantially water-soluble salt form, can be used as sequestering agents in a wide variety of detergent or laundry additive compositions.

Detergent compositions are generally a blend of a surfactant(s), builder(s) and, optionally, ion exchanger(s), filler(s), alkali(s), anticorrosion material(s), antiredeposition material(s), bleach(es), enzyme(s), optical brightener(s), fragrance(s) and other components selected for particular applications.

Detergent compositions incorporating the polymeric salt of the invention contain as essential components from 0.5% to 98% of a surfactant and from 2% to 99.5% of the polymeric compounds as a detergency builder, suitably in the sodium or ammonium salt form.

Preferably the detergent composition contains, in percent by weight of the composition: (a) 10-60% builder consisting of or including the polymeric compounds of the invention; (b) 2-25% surfactant; (c) optionally, 30% of other ingredients and a sufficient amount of water to either insure fluidity in the case of a liquid or to enhance processibility in the case of a solid granular or powdered detergent.

Surfactants that are useful in the present invention are the anionic (soap and non-soap), nonionic, zwitterionic and ampholytic compounds. The chemical nature of these detergent compounds is not an essential feature of the present invention. Moreover, such detergent compounds are well known to those skilled in the detergent art and the patent and printed literature are replete with disclosures of such compounds. Typical of such literature are "Surface Active Agents" by Schwartz and Perry and "Surface Active Agents and Detergents" by Schwartz, Perry and Berch, the disclosures of which are incorporated by reference herein.

The polymeric builder can be used either as the sole builder or, where desired, can be used in conjunction with other well-known builders, examples of which include water-soluble salts of phosphates, pyrophosphates, orthophosphates, polyphosphates, phosphonates, carbonates, carboxylates, polycarboxylates, succinates and the like.

In addition to the surfactant and builder, there may be optionally present additional ingredients which enhance the performance of the detergent composition. Typical examples of these include the well-known soil suspending agents, hydrotropes, corrosion inhibitors, dyes, perfumes, fillers, optical brighteners, enzymes, suds boosters, suds depressants, germicides, anti-tarnishing agents, cationic detergents, softeners, bleaches, buffers and the like. Other ingredients which may be optionally employed are such components as colouring dyes, suds stabilisers (eg dibutyl phthalate), fluorescent dyes, enzymes, perfumes, antiredeposition agents (eg carboxymethylcellulose), soil shield agents (eg hydropropyl methyl cellulose), bleaches, neutralised copolymers of ethylene and maleic anhydrides (eg EMA resins manufactured by the Monsanto company), co-surfactants and the like. Co-surfactants may be selected from the group consisting of anionic, cationic and zwitterionic surfactants and mixtures thereof. The bleaches may be chlorine or oxygen release types. The amounts of these additional ingredients or adjuvants may range from 0 to 30% by weight of the composition. Some of the adjuvants may be incorporated during the slurry preparation and others, because of stability considerations, may be post added to a spray-dried product as well known to those skilled in the art.

The detergent compositions of the present invention may be in any of the usual physical forms for such compositions, such as powders, beads, flakes, bars, tablets, noodles, liquids, pastes and the like.

The detergent compositions are prepared and utilised in the conventional manner. The wash solutions thereof desirably have a pH of 7 to 12, preferably 9 to 11 and contain typically 0.001 to 0.5% and preferably 0.009% to 0.3% by weight of the detergent composition and a corresponding amount of the polymeric compounds of this invention, preferably, for example, 0.005% to 0.3% by weight.

Synthesis of vinyl carbamate monomers

Vinyl carbamates may be prepared via the reaction of vinyl chloroformate and the corresponding amino acid. The HCl produced during the reaction may be neutralised by using at least one additional equivalent of amino acid or other base. Acetonitrile, dioxane/water, or other solvent systems may be used in the preparation of these novel vinyl carbamates. Reaction temperatures and times will, of course, vary for different amino acids.

Preparation of vinyl carbamate polymers

Vinyl carbamate copolymers may be prepared via the free radical polymerisation of a vinyl carbamate with an ethylenically unsaturated polymerisable comonomer.

Polymerisation of these vinyl carbamates is preferably accomplished in aqueous solution at temperatures of 5-85 °C, but preferably at temperatures of 25-65 °C, with concentrations varying from 1-80%. Polymerisations may be initiated with potassium persulphate but other initiators such as peroxides may also be used.

The following non-limiting Examples illustrate the invention.

All parts, percentages, and proportions herein and in the appended claims are by weight unless otherwise specified.

EXAMPLES

EXAMPLES 1-4: MONOMER SYNTHESIS

Example 1: Preparation of N-Vinyloxy Carbonyl (N-VOC) Aspartic Acid

Aspartic acid (20.0g, 0.150 mol) was added to a flask containing 180 ml of acetonitrile. Vinyl chloroformate (8.0g, 0.075 mol) was added to the mixture and the mixture was agitated. The mixture was refluxed with continued agitation for 2 hours and cooled to room temperature. The reaction mixture was then filtered. Solvent was removed from the filtrate in vacuo to yield N-VOC aspartic acid. The product was characterised by NMR ($^1$H and $^{13}$C) and mass spectrometry. $^1$H NMR (200 MHz, acetone-$d_6$) $\delta$2.96 (2H, dd, $-CH_2CO_2H$), 4.44, 4.71, 7.20 (3H, m, $CH_2=CH-$), 4.66 (1H, m, $-CH(CO_2H)$).

Example 2: Preparation of N-VOC Glutamic Acid

Glutamic acid (20.0g, 0.121 mol) was added to a flask containing 180 ml of acetonitrile. Vinyl chloroformate (6.4g, 0.060 mol) was added to the mixture and the mixture was agitated. The mixture was

refluxed for 2 hours with continued agitation and cooled to room temperature. The reaction mixture was then filtered. Solvent was removed from the filtrate in vacuo to yield N-VOC glutamic acid. The product was characterised by NMR ($^1$H and $^{13}$C) and mass spectrometry. $^1$H NMR (200 MHz, acetone-$d_6$) $\delta$2.08 (2H, m, $-CH_2-$), 2.39 (2H, t, $-CH_2CO_2H$), 4.20 (1H, m, $-CH(CO_2H)-$), 4.29, 4.57, 7.04 (3H, m, $CH_2=CH-$).

Example 3: Preparation of N-Vinyloxy Carbonyl (N-VOC) Glycine

Glycine (17.0g, 0.226 mol) was added to a flask containing 100 ml of acetonitrile. Vinyl chloroformate (12.5g, 0.117 mol) was added to the mixture and the mixture was agitated. The mixture was stirred at room temperature for 72 hours and filtered. Solvent was removed from the filtrate in vacuo to yield N-VOC glycine. The product was characterised by NMR ($^1$H and $^{13}$C) and mass spectrometry.

Example 4: Preparation of N-VOC Alanine

Alanine (20.0g 0.224 mol) is added to a flask containing 180 ml of acetonitrile. Vinyl chloroformate (12.0g, 0.112 mol) is added to the mixture and the mixture is agitated. The mixture is refluxed for 2 hours with continued agitation and cooled to room temperature. The reaction mixture is then filtered. Solvent is removed from the filtrate in vacuo to yield N-VOC Alanine. The product is characterised by NMR ($^1$H and $^{13}$C) and mass spectrometry.

EXAMPLES 5-8: POLYMER SYNTHESIS

A General Method for Preparing Poly(vinyl carbamates)

An aqueous solution of monomers (30-50% w/w) is sparged with a steady stream of nitrogen for 1 hour. Polymerisation is initiated with potassium persulphate (1% of the total weight of monomers). The solution is stirred at ambient temperatures and then heated to a range of about 35-70°C until an increase in viscosity is noted. The polymer is isolated by precipitating from a non-solvent.

Example 5: Preparation of Acrylic Acid/N-VOC Aspartic Acid Copolymer

Acrylic Acid (2.9g, 0.040 mol) and N-VOC aspartic acid (2.0g, 0.010 mol, Example 1) were dissolved in 25 ml of water. The solution was then sparged with a steady stream of nitrogen for 1 hour. Potassium persulphate (0.05g, $1.8 \times 10^{-4}$ mol) was then dissolved in the solution. The solution was stirred at 70°C for 16 hours to allow polymerisation. The polymer was then isolated by precipitation in acetonitrile. The precipitated polymer in turn was analysed by GPC and NMR ($^1$H and $^{13}$C). Nitrogen analysis of the polymer showed 11 molar % incorporation of the carbamate monomer. GPC analysis showed a molecular weight (Mw) of >750,000. Calcium binding studies of this polymer showed an upper log $K_{ca}$ value of 5.3 with unoccupied binding sites and a log $K_{ca}$ value of 4.5 with 50% of the binding sites occupied.

Example 6: Preparation of Maleic Acid/N-VOC Aspartic Acid Copolymer

Maleic Acid (3.0g, 0.026 mol) and N-VOC aspartic acid (5.2g, 0.026 mol, Example 1) were dissolved in 25 ml of water. The solution was then sparged with a steady stream of nitrogen for 1 hour. Potassium persulphate (0.08g, $3.0 \times 10^{-4}$ mol) was then dissolved in the solution. The solution was stirred at 70°C for 16 hours to allow polymerisation. The polymer was then isolated by precipitation in acetone. The precipitated polymer in turn was analysed by GPC and NMR ($^1$H and $^{13}$C). Nitrogen analysis of the polymer showed a 50 molar % incorporation of the carbamate monomer. GPC analysis showed a molecular weight (Mw) of 100,000. Calcium binding studies of this polymer showed an upper log $K_{ca}$ value of 6.5 with unoccupied binding sites and a log $K_{ca}$ value of 4.8 with 50% of the binding sites occupied.

Example 7: Preparation of Maleic Acid/N-VOC Glycine Copolymer

Maleic acid (9.6g, 0.083 mol) and N-VOC glycine (12.0g, 0.083 mol, Example 3) were dissolved in 54 ml of water. The solution was then sparged with a steady stream of nitrogen for 1 hour. Potassium persulphate (0.2g, $7.4 \times 10^{-4}$ mol) was then dissolved in the solution. The solution was stirred at 65°C for 16 hours to allow polymerisation. The polymer was then isolated by precipitation in acetone. The precipitated polymer in turn was analysed by GPC and NMR ($^1$H and $^{13}$C). Nitrogen analysis of the polymer showed a

50 molar % incorporation of each comonomer. GPC analysis showed a molecular weight (Mw) of 360,000. Calcium binding studies of this polymer showed an upper log $K_{ca}$ value of 6.1 with unoccupied binding sites and a log $K_{ca}$ value of 2.4 with 50% of the binding sites occupied.

Example 8: Preparation of Maleic Acid/N-VOC Alanine Copolymer

Maleic acid (10.0g, 0.086 mol) and N-VOC alanine (13.7g, 0.086 mol) are dissolved in 60 ml of water. The solution is then sparged with a steady stream of nitrogen for 1 hour. Potassium persulphate (0.2g, $7.4x10^{-4}$ mol) is then dissolved in the solution. The solution is stirred at 65°C for 16 hours to allow polymerisation. The polymer is then isolated by precipitation in acetone.

Determination of Calcium Binding

Calcium binding data was obtained at a pH of 10 by titrating 100 ml of 0.05g/l polymer solution at an ionic strength of 0.02-0.03 M (NaCl) with a 0.02 M $CaCl_2$ solution. A Radiometer (Trade Mark) calcium ion selective electrode was used to measure free $Ca^{++}$ ion concentration of the solutions. Data was corrected for dilution during each titration.

TABLE 1

| | 5 | | 6 | | A | | B | | C | |
|---|---|---|---|---|---|---|---|---|---|---|
| | Fraction Bound | Log $K_{ca}$ | Fraction Bound | Log $K_{ca}$ | Fraction Bound | Log $K_{ca}$ | Fraction Bound | Log $K_{ca}$ | Fraction Bound | Log $K_{ca}$ |
| 1 | 0 | 5.30 | 0 | 6.90 | 0 | 6.10 | 0 | 4.80 | 0 | 6.80 |
| 2 | 0.18 | 5.30 | 0.36 | 5.74 | 0.29 | 5.13 | 0.14 | 4.32 | 0.35 | 5.43 |
| 3 | 0.25 | 4.97 | 0.41 | 5.21 | 0.33 | 4.91 | 0.23 | 4.11 | 0.43 | 5.10 |
| 4 | 0.33 | 4.84 | 0.46 | 5.02 | 0.36 | 4.75 | 0.27 | 3.95 | 0.52 | 4.85 |
| 5 | 0.40 | 4.72 | 0.50 | 4.77 | 0.40 | 4.56 | 0.32 | 3.82 | 0.60 | 4.58 |
| 6 | 0.47 | 4.59 | 0.55 | 4.62 | 0.43 | 4.45 | 0.36 | 3.69 | 0.66 | 4.34 |
| 7 | 0.54 | 4.48 | 0.59 | 4.48 | 0.46 | 4.33 | 0.39 | 3.56 | 0.71 | 4.16 |
| 8 | 0.61 | 4.39 | 0.63 | 4.33 | 0.49 | 4.23 | 0.41 | 3.45 | 0.74 | 3.98 |
| 9 | 0.67 | 4.29 | 0.67 | 4.23 | 0.52 | 4.16 | 0.44 | 3.32 | 0.75 | 3.80 |
| 10 | 0.73 | 4.24 | 0.71 | 4.16 | 0.54 | 4.08 | 0.46 | 3.24 | 0.76 | 3.68 |
| 11 | 0.82 | 4.21 | 0.78 | 4.14 | 0.59 | 3.91 | 0.47 | 3.10 | | |

5 N-VOC aspartic acid/acrylic acid copolymer

6 N-VOC aspartic acid/maleic acid copolymer

A Builder U

B Polyacrylate

C Sokolan CP7

* Values obtained by extrapolation

TABLE 2

| | 7 | | A | | B | |
|---|---|---|---|---|---|---|
| | Fraction Bound | Log $K_{ca}$ | Fraction Bound | Log $K_{ca}$ | Fraction Bound | Log $K_{ca}$ |
| 1 | 0 | 6.10 | 0 | 6.10 | 0 | 4.80 |
| 2 | 0.25 | 5.47 | 0.29 | 5.13 | 0.14 | 4.32 |
| 3 | 0.29 | 5.02 | 0.33 | 4.91 | 0.23 | 4.11 |
| 4 | 0.33 | 4.81 | 0.36 | 4.75 | 0.27 | 3.95 |
| 5 | 0.37 | 4.66 | 0.40 | 4.56 | 0.32 | 3.82 |
| 6 | 0.41 | 4.52 | 0.43 | 4.45 | 0.36 | 3.69 |
| 7 | 0.45 | 4.39 | 0.46 | 4.33 | 0.39 | 3.56 |
| 8 | 0.48 | 4.26 | 0.49 | 4.23 | 0.41 | 3.45 |
| 9 | 0.52 | 4.16 | 0.52 | 4.16 | 0.44 | 3.32 |
| 10 | 0.55 | 4.04 | 0.54 | 4.08 | 0.46 | 3.24 |
| 11 | 0.58 | 3.95 | 0.59 | 3.91 | 0.47 | 3.10 |

7 N-VOC Glycine/Maleic acid copolymer
A Builder U
B Polyacrylate

* Values obtained by extrapolation

DESCRIPTION OF THE DRAWINGS

The Figures show log $K_{ca}$ values versus the fraction of bound sites along the polymer. As polymeric sequestrants chelate hardness ions, the binding of the polymer decreases. This decrease in binding capability is illustrated in the Figures which clearly show the binding of the novel polymeric carbamate builders to be excellent.

Figure 1 reports calcium binding data for the polymers of Examples 5 and 6 and other previously reported polymeric builders. Builder U (polyglyoxylic acid) (A) was obtained from Monsanto and had a molecular weight (Mw) of 8,000. Polyacrylic acid (B) had a molecular weight (Mw) of 60,000 and Sokolan (Trade Mark) CP7 (C) (a 2:1 copolymer of acrylic acid/maleic acid obtained from BASF) had a molecular weight of 52,000. The actual values are reported in Table 1.

Figure 2 reports calcium binding data for the polymer of Example 7 and other previously reported builders - Builder U (A) and polyacrylic acid (B). The actual values are reported in Table 2.

## Claims

1. A detergent composition comprising from 0.5% to 98% of a surfactant, and further comprising from 2% to 99.5% of a polymeric compound having a substantial water solubility and a log $K_{Ca}2+$ value greater than 4, the polymeric compound being a homo- or copolymer comprising from 5 to 100% of units of an N-vinyloxycarbonyl amino acid or salt selected from

N-vinyloxycarbonyl aspartic acid,
N-vinyloxycarbonyl glutamic acid,
N-vinyloxycarbonylglycine,
N-vinyloxycarbonylalanine,
N-vinyloxycarbonylaminobutyric acid,
N-vinyloxycarbonylcysteine,
N-vinyloxycarbonylornithine,
N-vinyloxycarbonyliminodiacetic acid,
and alkali metal or ammonium salts thereof,
and optionally from 0 to 95% of units of a comonomer which is selected from unsaturated carboxylic acids and their salts, esters and derivatives; and unsaturated dicarboxylic acids and their salts, esters, anhydrides and derivatives.

**2.** A detergent composition as claimed in claim 1, characterised in that the comonomer is selected from acrylic acid, maleic acid and maleic anhydride.

**3.** A detergent composition as claimed in claim 1 or claim 2, characterised in that the polymer has a molecular weight of from 1000 to 750 000.

**Patentansprüche**

**1.** Reinigungsmittelzusammensetzung, umfassend 0,5 bis 98% eines grenzflächenaktiven Mittels und des weiteren 2 bis 99,5% einer polymeren Verbindung mit einer merklichen wasserlöslichkeit und einem log $K_{ca}^{+2}$-Wert von mehr als 4, wobei die polymere Verbindung aus einem Homo- oder Copolymer mit 5 bis 100% Einheiten einer N-Vinyloxycarbonylaminosäure oder einem Salz, ausgewählt aus N-Vinyloxycarbonylasparaginsäure, N-Vinyloxycarbonylglutaminsäure, N-Vinyloxycarbonylglycin, N-Vinyloxycarbonylalanin, N-Vinyloxycarbonylaminobuttersäure, N-Vinyloxycarbonylcystein, N-Vinyloxycarbonylornithin, N-Vinyloxycarbonyliminodiessigsäure und den Alkalimetall- oder Ammoniumsalzen derselben, und gegebenenfalls 0 bis 95% Einheiten eines Comonomeren, ausgewählt aus ungesättigten Carbonsäuren und ihren Salzen, Estern und Derivaten sowie ungesättigten Dicarbonsäuren und ihren Salzen, Estern, Anhydriden und Derivaten, besteht.

**2.** Reinigungsmittelzusammensetzung nach Anspruch 1, dadurch gekennzeichnet, daß das Comonomer aus Acrylsäure, Maleinsäure und Maleinsäureanhydrid ausgewählt ist.

**3.** Reinigungsmittelzusammensetzung nach Anspruch 1 oder Anspruch 2, dadurch gekennzeichnet, daß das Polymer ein Molekulargewicht von 1000 bis 750.000 aufweist.

**Revendications**

**1.** Une composition détergente comprenant 0,5 % à 98 % d'un agent tensioactif, et comprenant en outre 2 % à 99,5 % d'un composé polymère présentant une solubilité dans l'eau substantielle et une valeur log $Ka_{Ca}^{2+}$ supérieure à 4, le composé polymère étant un homo- ou un copolymère comprenant 5 à 100 % d'unités d'un acide aminé ou d'un sel N-vinyloxycarbonyle sélectionné parmi :
de l'acide aspartique N-vinyloxycarbonyle,
de l'acide glutamique N-vinyloxycarbonyle,
de la N-vinyloxycarbonylglycine,
de la N-vinyloxycarbonylalanine,
de l'acide N-vinyloxycarbonylaminobutyrique,
de la N-vinyloxycarbonylcystéine,
de la N-vinyloxycarbonylornithine,
de l'acide N-vinyloxycarbonyliminodiacétique,
et des sels de métal alcalin ou d'ammonium de ceux-ci,
et, en option, 0 à 95 % d'unités d'un comonomère sélectionné parmi des acides carboxyliques insaturés et leurs sels, esters et dérivés ; et des acides dicarboxyliques insaturés et leurs sels, esters, anhydrides et dérivés.

2. Une composition détergente selon la Revendication 1, caractérisée en ce que le comonomère est sélectionné parmi de l'acide acrylique, de l'acide maléique et de l'anhydride maléique.

3. Une composition détergente selon la Revendication 1 ou la Revendication 2, caractérisée en ce que le polymère présente une masse moléculaire comprise entre 1 000 et 750 000.

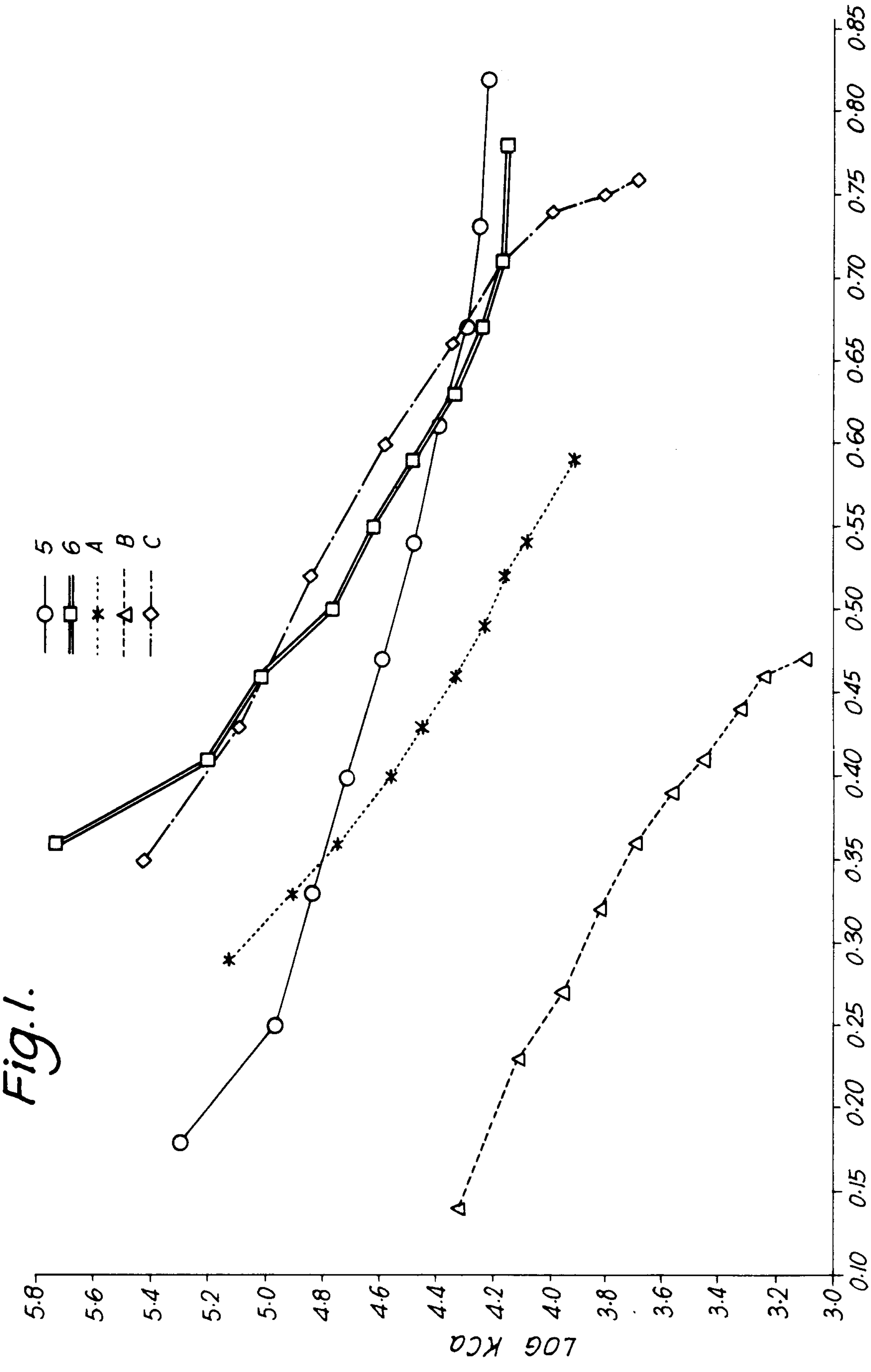
FRACTION BOUND
LOG Kca
5
6
A
B
C
0.10
0.15
0.20
0.25
0.30
0.35
0.40
0.45
0.50
0.55
0.60
0.65
0.70
0.75
0.80
0.85
3.0
3.2
3.4
3.6
3.8
4.0
4.2
4.4
4.6
4.8
5.0
5.2
5.4
5.6
5.8

Fig.1.

Fig.2.

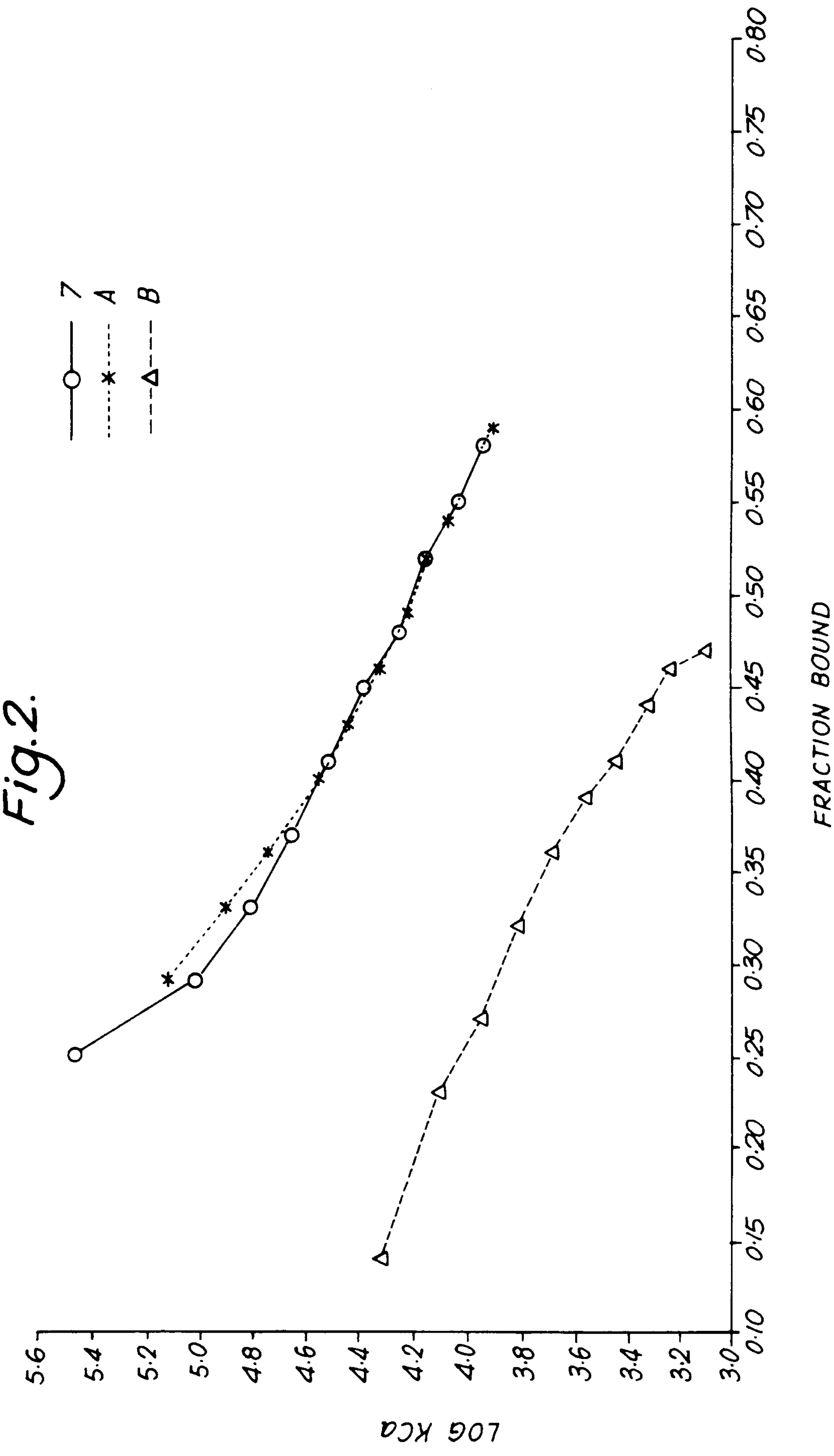

7
A
B
LOG KCa
5.6
5.4
5.2
5.0
4.8
4.6
4.4
4.2
4.0
3.8
3.6
3.4
3.2
3.0
FRACTION BOUND
0.10
0.15
0.20
0.25
0.30
0.35
0.40
0.45
0.50
0.55
0.60
0.65
0.70
0.75
0.80